# EUROPÄISCHE PATENTSCHRIFT

(11) **EP 0 963 734 B1**
(45) Veröffentlichungstag und Bekanntmachung des Hinweises auf die Patenterteilung: **27.02.2002**
(21) Anmeldenummer: 99111267.3
(22) Anmeldetag: 10.06.1999
(51) Int. Cl.: A61B 5/00, G08B 25/01

(54) **Vorrichtung zur Aufzeichung und Übertragung von medizinischen Daten**
Device for recording and transmission of medical data
Dispositif pour l'enregistrement et la transmission de données médicales

(30) Priorität: 10.06.1998 DE 19825898; 20.10.1998 DE 19848229
(43) Veröffentlichungstag der Anmeldung: 15.12.1999
(73) Patentinhaber: Ditec GmbH, 99097 Erfurt (DE)
(72) Erfinder: Lamster, Jörg, Dr., 99097 Erfurt (DE); Quinger, Matthias, 67122 Altrip (DE)
(74) Vertreter: Weidener, Jörg Michael

(56) Entgegenhaltungen:
- EP-A- 0 790 034
- DE-A- 4 441 907
- DE-A- 19 510 382
- US-A- 4 803 625
- US-A- 5 652 570

## Beschreibung

Die Erfindung bezieht sich auf ein am Körper eines Patienten tragbares mobiles Datenerfassungs- und Übertragungsgerät für medizinische Daten bzw. Meßgrößen, wie EKG, Blutdruck, Blutzucker, Sauerstoffsättigung des Blutes , Atmung , Temperatur, Atem- und Herzgeräusche, Herzfrequenz mittels Aufzeichnung und Übertragung von digitalisierten oder analogen Signalen.

Am Körper eines Patienten zu tragende EKG - Gerätesysteme sind seit einigen Jahren bekannt. Die EKG - Signale werden dabei meist über einen Zeitraum von 24 Stunden oder länger, in einem elektronischem Speicher, z.B. einem RAM , aufgezeichnet und zeitversetzt bzw. off-line über eine Schnittstelle am Monitor des behandelden Arztes ausgewertet. Rythmusstörungen am Herzen sind so nachträglich erkennbar.

Bekanntgeworden ist auch ein EKG - Fernübertragungssystem, bei dem die EKG - Signale mittels Tonfrequenzmodulation analog über das bekannte Festnetz - Telefon an einen Empfänger geleitet werden. Dieses System ermöglicht eine zeitnahe EKG - Überwachung eines Patienten , jedoch bei nur eingeschränkter Bewegungsfreiheit und einer zusätzlichen Mithilfe des Patienten durch Anwahl des Empfängers und Einleitung einer Übertragung. Das übertragene EKG entspricht so einerseits nicht dem normalem freien Tagesablauf des Patienten und ist andererseits umständlich handhabbar.

Mit der DE 196 46 603 ist auch ein GPS - Handy bekannt geworden , dessen GPS- Empfänger (Global Positioning System) GPS-Satelliten-Signale empfängt und Positionsdaten des Handys bzw. Handy-Benutzers auf einem Display zur Anzeige bringt und mittels Handbefehl die ermittelten Standortkoordinaten per Funk an eine Station überträgt . Nachteil des Gerätes ist, daß lediglich die Position des Gerätes ermittelt werden kann.

Aus der EP-A-0 790 034 ist eine Vorrichtung bekannt, die die Merkmale des ersten Teils des Anspruchs 1 aufweist.

Der Erfindung liegt daher die Aufgabe zugrunde , eine Vorrichtung der eingangs genannten Art zu schaffen , die die vollautomatische und Online - EKG - Überwachung und die Überwachung anderer medizinischer Meßgrößen , wie Blutdruck, Blutzucker, Sauerstoffsättigung des Blutes, Atmung , Temperatur, Körpergeräusche, Körperlage, Herzfrequenz eines Patienten unabhängig von dessen Aufenthaltsort und ohne dessen Mitwirkung gestattet und dessen Position und Notlage feststellt .

Diese Aufgabe wird erfindungsgemäß dadurch gelöst, daß ein am Körper eines Patienten tragbares medizinisches Datenerfassungsgerät mit Analysesoftware für EKG und mögliche weitere Meßgrößen wie, Blutdruck, Sauerstoffsättigung des Blutes , Atmung, Temperatur mit den Hauptbaugruppen AD-Wandler, elektronischer Speicher, Mikrocontroler, mit einem Telefonschaltkreis und einem Sende-Empfangs- Schaltkreis eines Mobiltelefons für ein Funknetz verbunden ist, so daß die digitalisierten Signale der Herzstromkurven des AD-Wandlers, sowie die Signale für Blutdruck , Blutzucker, Atmung , Temperatur und weiterer Meßgrößen, sowohl in den RAM-Speicher des Datenerfassungs-Gerätes, als auch über ein Funknetz (z.B. D-Netz) wahlweise on-line, zeitversetzt oder im Falle einer vom Gerät erkannten Störung an eine externe Empfangsstation übertragen und dort zeitgleich ausgewertet oder aufgezeichnet werden. Es erkennt der Mikrocontroler der EKG-Einheit eine Rhythmusstörung per Analysesoftware und löst in diesem Fall selbständig die Übertragung des im Loop-Speicher der EKG-Einheit gespeicherten EKG-files aus. Diese Daten können auch zu beliebigen Zeiten von der Überwachungs- bzw. Empfangsstation abgerufen werden. Rhytmusstörungen werden so rechtzeitig erkannt und können dem Patienten signalisiert werden. Bei bedrohlichen Rhytmusstörungen kann so rechtzeitig der medizinische Notdienst alarmiert werden. Zwischenzeitliche Arztbesuche sind nicht mehr erforderlich und der Patient kann sich während der Aufzeichnung und während einer Übertragung frei bewegen.

Gleichzeitig weist das modulare Datenerfassungs- und Übertragungsgerät einen GPS-Schaltkreis (Global Positioning-System) auf, der Satelliten-Signale empfängt und mit diesen die Position des mobilen Gerätes feststellt und der Basisstation, z.B. eine Rettungsstelle, per Funk überträgt.

Da der GPS-Empfänger in umbauten Räumen kaum empfangsfähig ist , wird in festgelegten zeitlichen Abständen (z.B. minütlich) der GPS - Eingang abgefragt . Sobald sich der Träger des Gerätes mit dem GPS-Modul im Freien befindet , werden die empfangenen Positionsdaten gespeichert und gleichzeitig die vorhergehenden Positionsdaten gelöscht . Findet der Eingang kein Signal, so erfolgt keine Speicherung. Damit sind die Positionsdaten abrufbar, die den Standort in unmittelbarer Nähe des umbauten Raumes repräsentieren. Das Verfahren dient gleichzeitig zur Energieeinsparung .

Die Verbindung des elektronischen Speichers des mobilen medizinischen Datenerfassungsgerätes mit dem Mobil - Telefon-Schaltkreis und dem GPS-Empfänger gestattet sowohl die Aufzeichnung der EKG-und anderen medizinischen Signale im elektron. Speicher, als auch die gleichzeitige On-line -'Übertragung zur externen Empfangsstation, z.B. beim Arzt, mit digitaler Aufzeichnung und/oder einer echtzeitlichen Auswertung über Monitor oder durch Anschluß eines Druckers .
Bei bedrohlichen Situationen kann jederzeit die geografische Position des Trägers des Gerätes ermittelt werden.

Findet der Sende- Empfangs-Schaltkreis des Mobiltelefon-Modems keinen Anschluß im D-Netz, so erfolgt in festgelegten zeitlichen Abständen über eine automatische WAHLWIEDERHOLUNG eine erneute Übertragung , oder die Übertragung wird automatisch auf eine festgelegte andere Telefon-Nr. geleitet.

Nach erfolgter Übertragung kann der elektronische Speicher vom Empfänger über ein Steuersignal gelöscht werden .

Der der Erfindung zugrundeliegende Gedanke wird in einer nachfolgenden Beschreibung anhand eines Ausführungsbeispieles , das in der Zeichnung dargestellt ist, näher erläutert.

Dabei zeigt die
- Fig.1: ein Blockschaltbild der erfindungsgemäßen Vorrichtung mit Telefon-schaltkreis und mit einem GPS-Empfänger;
- Fig. 2: ein typisches auswertbares EKG-Signal;

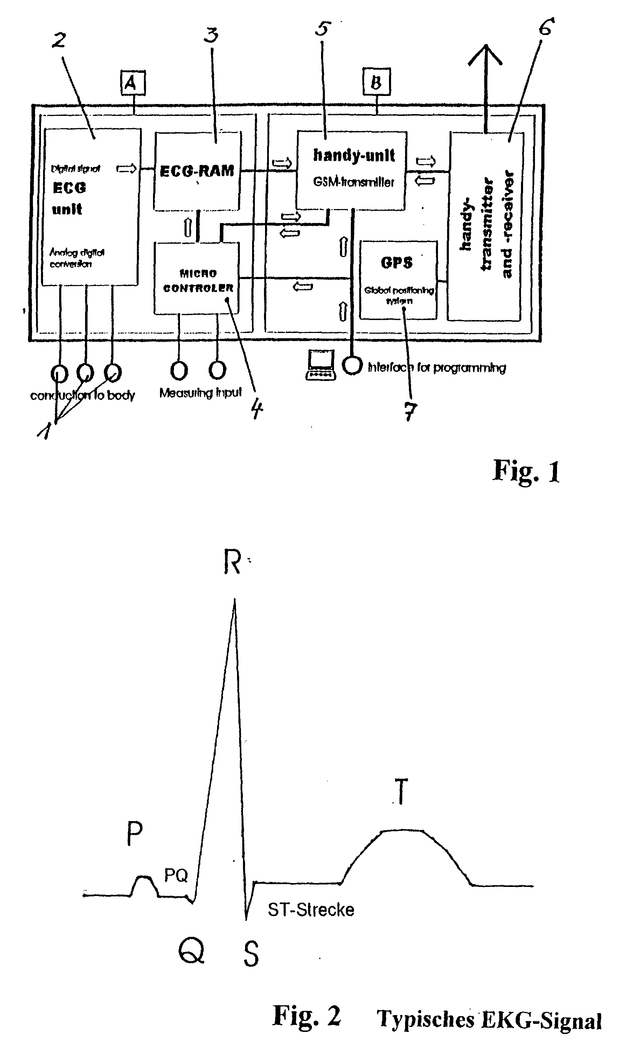

Die von den Elektroden 1 (Fig.1) aufgenommenen analogen Signale , z.B. Herzstromkurven, werden im AD-Wandler 2 des EKG-Gerätes (A) umgewandelt und digital im RAM-Speicher 3 gespeichert. Der Microcontroler 4 sorgt für eine Analyse der erfaßten Werte und eine Steuerung der erfassten Signale und ist mit dem elektronischem Speicher 3 und mit dem Telefon-Schaltkreis 5 des Mobiltelefons (B) verbunden . Zur Programmierung und für das LESEN des Inhaltes des Speichers 3 weist der Telefon- Schaltkreis 5 ein Interface für einen PC auf. Für das SENDEN und EMPFANGEN von digitalen Signalen ist der Telefon-Schaltkreis 5 mit einem Sende-Empfänger-Schaltkreis 6 des Mobiltelefons(B) verbunden .
Die erfindungsgemäße Vorrichtung ist somit für das Speichern erfasster Signale in einem Speicher, z.B. RAM, und für das Senden digitaler Daten in ein Funknetz geeignet.

Das typische EKG-Signal (Fig. 2) besteht aus folgenden wesentlichen aufeinander folgenden Komponenten - die P-Welle, der QRS-Komplex und die T-Welle. Zwischen der P-Welle und dem QRS-Komplex liegt in der isoelektrischen Ebene die PQ-Strecke. Zwischen dem QRS-Komplex urid der T-Welle liegt die für diagnostische Kriterien wichtige ST-Strecke.

Optimal werden die gespeicherten Daten zu einer gewünschten oder einer vorher festgelegten Zeit per Funktelefon-Netz an einen Empfänger übertragen oder vom Empfänger abgerufen . Die Übertragung der digitalen Daten kann aber auch zeitgleich mit der Erfassung der analogen Signale am Patienten erfolgen, wobei der Empfang an einer externen Empfangsstation on-line an einem Monitor erfolgt bzw. digital, z. B. auf einer Festplatte des angeschlossenen PC's gespeichert wird oder zeitgleich über einen Drucker zur Ausgabe kommt.

Die im Speicher des EKG-Gerätes (B) bzw. elektronischen Datenerfassungsgerätes etablierte Software erkennt Rhytmusstörungen selbständig und löst die automatische Übertragung der EKG-Episode aus .

Das gleiche gilt für andere Meßgrößen , wie z.B. die Sauerstoffsättigung oder den Blutdruck, wobei im Falle einer Schwellwert-Überschreitung eine automatische Übertragung der Meßgrößen, der Uhrzeit und wahlweise der geografischen Position erfolgt .

Bei bedrohlichem Zustand des Patienten , z. B. bei Vorliegen bestimmter Schwellenwerte, werden die über GPS ermittelten Postionsdaten mit an die Basisstation übermittelt.

Die Übertragungsmöglichkeit von digitalen Signalen über ein Funk-Netz ermöglicht auch die Fernsteuerung des EKG-und medizinischen Datenerfassungsgerätes, z.B. die Umprogrammierung, aber auch die Erfassung und Übertragung solcher Meßgrößen, wie Blutdruck, Blutzucker, Sauerstoffsättigung des Blutes, Atmung, Temperatur des angeschlossenen Patienten.

Weiterhin weist das am Körper des Patienten zu tragende Gerät einen Lagesensor auf, dessen über Sender 6 übertragenen Daten über "stehend", "liegend-oben" " liegend-unten" Aufschluß gibt.

Die erfindungsgemäße Vorrichtung hat so den Vorteil , daß sie die life -Überwachung eines Patienten gestattet, eine sofortige Alarmierung im Falle eines bedrohlichen Zustandes ermöglicht und die geografische Position des Patienten feststellen kann.

## Patentansprüche

1. Vorrichtung zur Aufzeichnung und Übertragung von medizinischen Daten, wie EKG - Signale und Meßwerte, in funktioneller Verbindung der Hauptbaugruppen Körperelektroden (1), Speicher (3), Mikrocontroller (4) mit einem A/D-Wandler (2), einem Funktelefon-Modem (B) mit den Baugruppen des Telefon-Schaltkreises (5) mit PC- Schnittstelle, Sendempfangs-Schaltkreis (6), in einem am Körper einer Person zu tragenden elektronischen Datenerfassungsgerätes mit on-line -Überwachung, **dadurch gekennzeichnet, daß** die vom Patienten abgeleiteten EKG-Signale mit deren typischen Komponenten P-Welle, QRS-Komplex und T-Welle im Mikrocontroller (4) mit Hilfe einer Analysesoftware einer Analyse unterzogen werden, wobei kritische von der Norm abweichende EKG-Abschnitte die Funkverbindung zwischen dem mobilen Gerät und einer Überwachungsstation schalten und die von der Norm abweichenden EKG-Abschnitte und Rhythmusstörungen mit den zeitlich vorangehenden im Speicher abgelegten Signalen per Mobilfunk an die Überwachungsstation gesendet und dort aufgezeichnet werden.

2. Vorrichtung nach Anspruch 1, **dadurch gekennzeichnet, daß** wahlweise unter der Bedingung einer von einem Lagesensor erfaßten abnormen Körperlage sofort eine Übertragung an die Überwachungsstation erfolgt.

3. Vorrichtung nach Anspruch 1 und 2, **dadurch gekennzeichnet, daß** bei Aktivierung des Funktelefons die über ein GPS (7) ermittelten geografischen Positionsdaten übertragen werden.

4. Vorrichtung nach Anspruch 1 bis 3, **dadurch gekennzeichnet, daß** wahlweise eine Änderung der Einstellungen und des Programmes des Gerätes von der Überwachungsstation erfolgt.

5. Vorrichtung nach Anspruch 1, **dadurch gekennzeichnet, daß** der Telefonschaltkreis (5) des Funktelefons (B) mit einer Wahlwiederholung ausgestattet ist und der SendeEmpfänger- Schaltkreis (6) des Funktelefons (B) nach erfolgter Übertragung ein Quitt - Signal erhält .

6. Vorrichtung nach Anspruch 1 bis 5, **dadurch gekennzeichnet, daß** eine Not-Taste für die aktive Auslösung der Übertragung angeordnet ist.

7. Vorrichtung nach Anspruch 1 bis 6, **dadurch gekennzeichnet, daß** eine integrierte Schallwandler-Einheit zum Senden und Empfangen sprachlicher Signale angeordnet ist.

8. Vorrichtung nach Anspruch 1 bis 7, **dadurch gekennzeichnet, daß** weitere Sensoren wie zur Messung der Sauerstoffsättigung des Blutes, Blutdruck, Körpertemperatur, Atemtätigkeit, Körpergeräusche, wie Atmungs- und Herzgeräusche, Körperlage , Herzfrequenz, Blutzucker angeordnet sind, wobei der Lagesensor eine abnorme Körperlage über einen längeren Zeitraum registriert und einen Notfall signalisiert.

## Claims

1. A device for recording and transmitting medical data such as ECG signals and measurements made by operatively connected main sub-assemblies, i.e. body electrodes (1), a memory (3) and a microcontroller (4) with an A/D converter (2), a radio-telephone unit (B) and the sub-assemblies of the telephone circuit (5) comprising a PC interface and a transmitting and receiving circuit (6) in an electronic data acquisition device for wearing on the body and with on-line monitoring, **characterised in that** the typical components (P wave, QRS complex and T wave) of the ECG signals derived from the patient are analysed in the microcontroller (4), using analytical software, wherein critical abnormal ECG portions make a radio connection between the mobile device and a monitoring station and the abnormal ECG portions and rhythm disturbances, together with the signals previously deposited in the memory, are transmitted by mobile radiotelephone to the monitoring station and are recorded therein.

2. A device according to claim 1, **characterised in that** an immediate transmission to the monitoring station can be made if an abnormal body position is detected by a position sensor.

3. A device according to claims 1 and 2, **characterised in that** when the radio telephone is activated, the geographical position data acquired via a GPS (7) are transmitted.

4. A device according to claims 1 to 3, **characterised in that** the adjustments and program of the device can if required be altered by the monitoring station.

5. A device according to claim 1, **characterised in that** the circuit (5) of the radio telephone (B) is equipped with a repeat last call means, and the transmitter-receiver circuit (6) of the radio telephone (B) receives an acknowledgement signal after transmission.

6. A device according to claim 1 to 5, **characterised in that** an emergency key for actively triggering the transmission is provided.

7. A device according to claims 1 to 6, **characterised in that** an integrated sound-converter unit is provided for transmitting and receiving voice signals.

8. A device according to claims 1 to 7, **characterised in that** additional sensors, e.g. for measuring the saturation of the blood with oxygen, the blood pressure, the body temperature, respiration, body noises such as breathing and heart noises, body position, heart frequency or blood sugar are provided, the position sensor recording an abnormal body position for a prolonged period and indicating an emergency.

## Revendications

1. Dispositif pour enregistrer et transmettre des données médicinales, comme des signaux E.C.G. et des valeurs de mesure dans une liaison fonctionnelle des modules principaux : électrodes corporelles (1), mémoire (3), microcontrôleur (4) avec un convertisseur analogique-numérique (2), une unité de radiotéléphone (B) avec les modules du circuit de commutation téléphonique (5) avec interface PC, circuit de commutation émission-réception (6) dans une unité électronique d'acquisition de données équipée d'une surveillance en ligne et à porter sur le corps d'une personne, **caractérisé en ce que** les signaux E.C.G. provenant du patient avec leurs composants typiques ondes P, complexe QRS et onde T dans le microcontrôleur (4) sont soumis à une analyse à l'aide d'un logiciel d'analyse, dans lequel des sections E.C.G critiques s'écartant de la norme commutent la liaison entre l'appareil mobile et un poste de surveillance, et les sections E.C.G s'écartant de la norme et les perturbations rythmiques avec les signaux survenus auparavant stockés en mémoire sont envoyées par le radiotéléphone au poste de surveillance et y sont enregistrées.

2. Dispositif selon la revendication 1, **caractérisé en ce qu'**une transmission au poste de surveillance ait lieu immédiatement au choix sous la condition d'une position corporelle hors norme détectée par un capteur de position.

3. Dispositif selon les revendications 1 et 2, **caractérisé en ce que** des données de position géographiques détectées par un GPS (7)(système de positionnement global) sont transmises lors de l'activation du radiotéléphone.

4. Dispositif selon les revendications 1 à 3, **caractérisé en ce qu'**au choix une modification des paramètres et du programme de l'appareil se fait par le poste de surveillance.

5. Dispositif selon la revendication 1, **caractérisé en ce que** le circuit téléphonique (5) du radiotéléphone (B) est équipé d'une recomposition automatique de numéro et que le circuit de commutation émission-réception (6) du radiotéléphone (B) reçoit un signal d'acquittement après une transmission ayant réussi.

6. Dispositif selon les revendications 1 à 5, **caractérisé en ce qu'**une touche d'urgence est disponible pour le déclenchement actif de la transmission.

7. Dispositif selon les revendications 1 à 6, **caractérisé en ce qu'**une unité intégrée de conversion sonore est disponible pour émettre et recevoir des signaux vocaux.

8. Dispositif selon les revendications 1 à 7, **caractérisé en ce que** sont disposés d'autres capteurs comme pour mesurer la saturation en oxygène du sang, la pression artérielle, la température du corps, l'activité respiratoire, des bruits corporels comme des bruits de respiration et de battements cardiaques, la position du corps, la fréquence cardiaque, la glycémie, le capteur de position enregistrant une position anormale du corps pendant une période prolongée et signalant un cas d'urgence.
